(19) Europäisches Patentamt
European Patent Office
Office européen des brevets

(11) Publication number: **0 200 345 A2**

# (12) EUROPEAN PATENT APPLICATION

(21) Application number: **86302138.2**

(22) Date of filing: **24.03.86**

(51) Int. Cl.[4]: **C07D 263/58**, C07C 91/44, C07C 121/84, C07C 121/78, C07D 307/52, C07D 333/20, C07C 97/10, A61K 31/135, A61K 31/275, A61K 31/42, A61K 31/34

(30) Priority: **30.03.85 GB 8508375**
**07.09.85 GB 8522241**

(43) Date of publication of application:
**10.12.86 Bulletin 86/45**

(84) Designated Contracting States:
**BE CH DE FR GB IT LI NL**

(71) Applicant: **BEECHAM GROUP PLC**
**Beecham House Great West Road**
**Brentford Middlesex TW8 9BD(GB)**

(72) Inventor: **Buckle, Derek Richard**
**18 Hillfield Road**
**Redhill Surrey, RH1 4AP(GB)**
Inventor: **Webster, Richard Andrew Bentley**
**86 Surrey Grove**
**Sutton Surrey, SM1 3PN(GB)**

(74) Representative: **Blake, John Henry Francis et al**
**Beecham Pharmaceuticals Great Burgh Yew Tree Bottom Road**
**Epsom, Surrey KT18 5XQ(GB)**

(54) **Anti-allergic or anti-inflammatory substituted (hetero)-aralkylamino-ortho-phenols.**

(57) Compounds of the general formula (I)

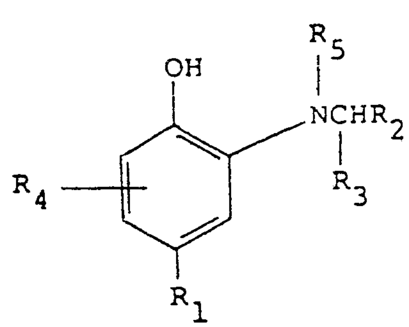

(I)

EP 0 200 345 A2

or a pharmaceutically acceptable salt or solvate thereof;

wherein $R_1$ is straight or branched $C_{1-6}$ alkyl, halo, nitro, cyano, hydroxy, or $COR_6$ wherein $R_6$ is straight or branched $C_{1-6}$ alkyl or aryl,

$R_2$ is substituted or unsubstituted aryl, aralkyl or heteroaryl;

$R_3$ is hydrogen or straight or branched $C_{1-6}$alkyl;

$R_4$ is hydrogen, hydroxy, straight or branched $C_{1-6}$ alkyl, halo, nitro, cyano, $C_{1-6}$ alkoxy, halo($C_{1-6}$)alkyl; amino, carboxylic ester, carboxylic acid, $COR_6$,$SR_6$, $SO_2R_6$, $NHCOR_6$ or $NHR_6$ wherein $R_6$ is as defined above; and

$R_5$ is hydrogen, straight or branched $C_{1-6}$ alkyl, substituted or unsubstituted benzyl or $COR_6$ wherein $R_6$ is as defined above;

are of value in the prophylaxis and treatment of

allergic and inflammatory disorders.

# CHEMICAL COMPOUNDS

The present invention relates to substituted aminophenols, to processes for preparing them, to pharmaceutical compositions containing them and their use in medicine.

Japanese Published Patent Application (Kokai) No. 81-02976 discloses compounds of formula (A)

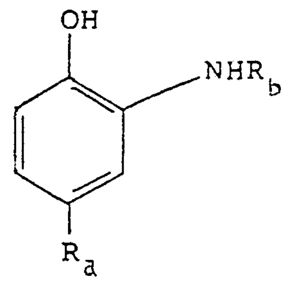

(A)

in which inter alia $R_a$ is $CH_3$, Cl or $NO_2$, and $R_b$ is $-CH_2C_6H_5$. These compounds are indicated solely as intermediates in the preparation of pharmacologically active benzoxazines.

It has been found that a class of aminophenol derivatives, certain of which are novel, are active as inhibitors of the enzyme 5-lipoxygenase, an important enzyme in the production of the spasmogenic peptide leukotrienes($LTC_4$, $LTD_4$, $LTE_4$) and inflammatory leukotriene $LTB_4$. These compounds are also active at inhibiting SRS-A production in vivo and are thus of value in the prophylaxis and treatment of diseases whose symptons are controlled by these mediators such as asthma, rheumatoid arthritis, psoriasis and other allergic and inflammatory disorders.

According to the present invention there is provided a pharmaceutical composition comprising a compound of formula (I)

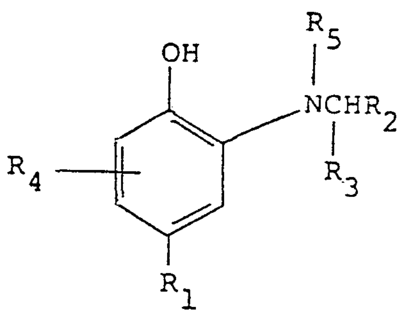

(I)

or a pharmaceutically acceptable salt or solvate thereof;

wherein $R_1$ is straight or branched $C_{1-6}$ alkyl, halo, nitro, cyano, hydroxy, or $COR_6$ wherein $R_6$ is straight or branched $C_{1-6}$ alkyl or aryl,

$R_2$ is substituted or unsubstituted aryl, aralkyl or heteroaryl;

$R^3$ is hydrogen or straight or branched $C_{1-6}$alkyl;

$R_4$ is hydrogen, hydroxy, straight or branched $C_{1-6}$ alkyl, halo, nitro, cyano, $C_{1-6}$ alkoxy, halo($C_{1-6}$)alkyl; amino, carboxylic ester, carboxylic acid, $COR_6$,$SR_6$, $SO_2R_6$, $NHCOR_6$ or $NHR_6$ wherein $R_6$ is as defined above; and

$R_5$ is hydrogen, straight or branched $C_{1-6}$ alkyl, substituted or unsubstituted benzyl or $COR_6$ wherein $R_6$ is as defined above;

and a pharmaceutically acceptable carrier.

As used herein the term "aryl" includes phenyl. Hetero aryl groups are typically 4 to 7 membered rings containing up to four heteroatoms selected from oxygen, sulphur and nitrogen.

When $R_1$ is $C_{1-6}$ alkyl, it is preferably methyl, iso-propyl or tert-butyl. As $COR_6$ it may be for example acetyl or benzyl. Bromo or chloro are typical halo-substituents for $R_1$.

Suitably $R_2$ is substituted or unsubstituted phenyl. As an heteroaryl group it may be, for exmaple, furan, thiophene, imidazole, isoxazole or pyridine and be substituted or unsubstituted. A suitable aralkyl group, which may also be substituted or unsubstituted, is benzyl.

- Suitable optional substituents for $R_2$ are one or more groups selected from straight or branched $C_{1-6}$ alkyl, hydroxy, halo, nitro, cyano, $C_{1-6}$ alkoxy, halo ($C_{1-6}$) alkyl, amino, carboxylic ester, carboxylic acid, $COR_6$, $NHCOR_6$, $NHR_6$, $N(R_6)_2$, $SR_6$, $SOR_6$ or $SO_2R_6$ where $R_6$ is as hereinbefore defined.

Preferred substituents for $R_2$ are $C_{1-6}$ alkyl, halo, nitro, cyano, alkoxy such as methoxy, halo($C_{1-6}$)alkyl such as trifluoromethyl, amino, carboxylic ester, carboxylic acid, $COR_6$ or $NHCOR_6$ wherein $R_6$ is as hereinbefore defined.

Suitable carboxylic ester groups include $-CO_2R_6$ where $R_6$ is as hereinbefore defined.

$R_2$ substituents are typically meta or para with respect to the $-CH_2$-linking group, preferably para.

Suitably $R_3$ is hydrogen, or methyl when $C_{1-6}$ alkyl.

A suitable halo($C_{1-6}$) alkyl group for $R_4$ is trifluoromethyl. As $C_{1-6}$ alkyl $R_4$ is typically methyl or tert butyl, as $COR_6$ it is typically benzoyl. When $R_4$ is other than hydrogen, it is preferably in the 6-position.

Preferably $R_4$ is hydrogen.

A suitable acyl $COR_6$ group for $R_5$ is $-COCH_3$.

Preferably $R_5$ is hydrogen.

$R_6$ is typically methyl, ethyl, or phenyl.

Within formula (I) there is a group of compounds in which:

$R_1$ is methyl, ethyl, iso-propyl, tert -butyl, benzoyl, chloro or bromo,

$R_2$ is phenyl, benzyl, furyl or thienyl, optionally substituted by methyl, iso-propyl, cyano, methoxy, chloro, bromo, fluoro, trifluoromethyl, hydroxy or nitro, $R_3$ is hydrogen or methyl,

$R_4$ is hydrogen or hydroxy, and

$R_5$ is hydrogen or benzyl.

Examples of compounds of formula (I) include:

2-phenylmethylamino-4-chlorophenol, 2-phenylmethylamino-4-methylphenol, and salts and solvates thereof.

Certain compounds of formula (I) are novel.

Therefore further according to the present invention there is provided a compound of formula (IA):

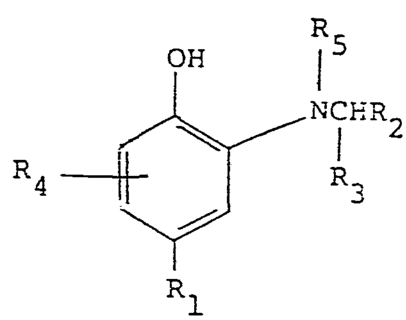

(IA)

wherein $R_1$, $R_2$, $R_3$, $R_4$ and $R_5$ are as defined in relation to formula (I), provided that when $R_1$ is methyl or chloro, $R_2$ is phenyl, $R_3$ is hydrogen and $R_4$ is hydrogen, $R_5$ is other than hydrogen.

Suitable and preferred substituents are as described for formula (I) where appropriate.

When compounds of formula (IA) form salts (such as acid addition salts) or solvates (such as hydrates) these also form part of this invention.

The compounds of formula (IA) or their salts or solvates are preferably in pharmaceutically acceptable or substantially pure form.

By pharmaceutically acceptable form is meant, inter alia, of a pharmaceutically acceptable level of purity excluding normal pharmaceutical additives such as diluents and carriers, and including no material considered toxic at normal dosage levels.

A substantially pure form will generally contain at least 50% (excluding normal pharmaceutical additives), preferably 75%, more preferably 90% and still more preferably 95% of the compound of formula I or its salt or solvate.

One preferred pharmaceutically acceptable form is the crystalline form.

Salt or solvates of compounds of formula (IA) which are not pharmaceutically acceptable, may be useful as intermediates to prepare pharmaceutically acceptable compounds of the invention.

Suitable pharmaceutically acceptable salts of the invention are acid addition salts.

Suitable acid addition salts of the compound of formula (IA) include pharmaceutically acceptable inorganic salts such as the sulphate, nitrate, phosphate, borate, hydrochloride and hydrobromide and pharmaceutically acceptable organic acid addition salts such as acetate, tartrate, maleate, citrate, succinate, benzoate, ascorbate, methanesulphate, α-keto glutarate, α-glycerophosphate, and glucose-1-phosphate. Preferably the acid addition salt is a hemisuccinate, hydrochloride, α-ketoglutarate, α-glycerophosphate or glucose-1-phosphate, in particular the hydrochloride salt.

Examples of compounds of formula (IA) include:

2-[(4-cyanophenyl)methyl]amino-4-methylphenol;

2-[(4-methylphenyl)methyl]amino-4-methylphenol

2-[(4-methoxyphenyl)methyl]amino-4-methylphenol;

2-[(4-chlorophenyl)methyl]amino-4-methylphenol;

2-[(2-furyl)methyl]amino-4-methylphenol;

2-[(3-trifluoromethylphenyl)methyl]amino-4-methylphenol;

2-[(4-fluorophenyl)methyl]amino-4-methyl phenol;

2-[(3,4 dichlorophenyl)methyl]amino-4-methyl phenol;

2-[(thien-2-yl)methyl]amino-4-methyl phenol;

2-[(4-bromophenyl)methyl]amino-4-methyl phenol;

2-[(3-fluorophenyl)methyl]amino-4-methyl phenol;

2-phenylmethylamino-4-iso-propylphenol;

2-phenylmethylamino-4-tert-butylphenol;

4-benzoyl-6-(phenylmethylamino) resorcinol;

2-dibenzylamino-4-methyl phenol;

2-phenylmethylamino-4-chlorophenol;

2-phenylmethylamino-4-bromophenol;

2-phenylmethylamino-4-ethylphenol;

2-[)4-nitrophenyl]methyl]amino-4-methylphenol;

2-](4-iso-propylphenyl)methyl]amino-4-methylphenol;

2-[(4-hydroxyphenyl)methyl]amino-4-methylphenol;

2-phenylethylamino-4-methylphenol;

and salts and solvates thereof.

The present invention also provides a process for preparing compounds of formula (I) and (IA) wherein $R_5$ is hydrogen by hydrolysis of a compound of formula (II)

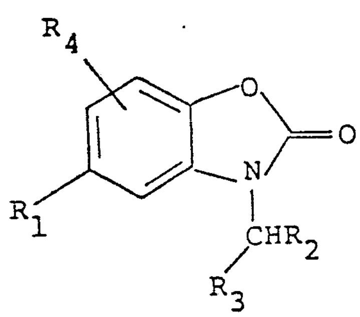

(II)

wherein $R_1$, $R_2$, $R_3$ and $R_4$ are as defined in relation to formula (I).

The hydrolysis reaction is suitably carried out in the presence of an excess of base (such as potassium or sodium hydroxide) in an organic solvent (e.g. methanol). The reaction is performed at an elevated temperature of from 30°C to 100°C under reflux.

Compounds of formula (II) can be prepared by reaction of the compound of formula (III)

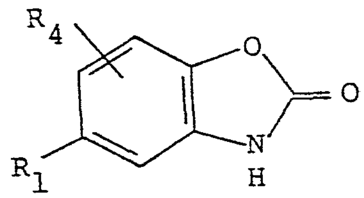

(III)

wherein $R_1$ and $R_4$ are as defined in relation to formula (I); with a compound of formula (IV)

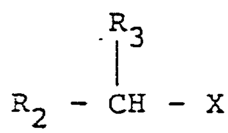

(IV)

wherein $R_2$ and $R_3$ are as defined in relation to formula (I) and X is halogen, such as chlorine, bromine or iodine.

The reaction is suitably carried out in an inert solvent (such as a mixture of ethanol and water) in the presence of a base. Suitable bases include sodium hydroxide and potassium hydroxide. The reaction is carried out at an elevated temperature of from 60°C-100°C under reflux.

The compound of formula (III) can be prepared by reaction of a compound of formula (V)

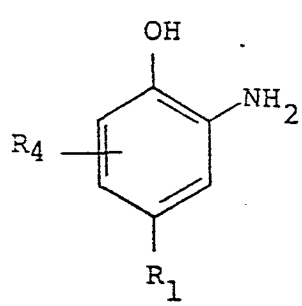

(V)

wherein $R_1$ and $R_4$ are as defined in relation to formula (I) with urea.

The reaction is suitably carried out at elevated temperatures of from 135°C to 320°C, preferably at 160°C, for a period of 2-3 hours.

Compounds of formulae (IV) and (V) are either known compounds or can be prepared from known compounds by known methods.

The present invention further provides a process for preparing compounds of formula (I) or (IA) wherein $R_5$ is hydrogen by reduction of a compound of formula (VI)

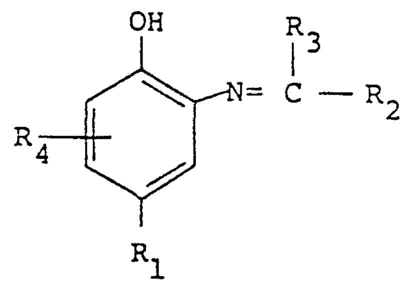

(VI)

wherein $R_1$, $R_2$, $R_3$ and $R_4$ are as defined in relation to formula (I).

Suitable reducing agents include hydrogen in the presence of a catalyst such as palladium, or hydride reducing agents. The particular hydride reagent employed can be selected in accordance with the nature of $R_1$, $R_2$, $R_3$ and $R_4$. Examples of such reagents are $NaBH_3CN$, $B_2H_6$ etc.

The reaction will be carried out in an organic solvent such as ethanol and if necessary at a low pH for example at pH 1-2.

Alternatively $LiA1H_4$ may suitably be used in ethereal solvents such as diethyl ether or tetrahydrofuran.

Moderate temperature of from 0°C to 60°C, conveniently ambient temperature, will be employed.

Compounds of formula (VI) can be prepared by reaction of a compound of formula (V) as hereinbefore defined, with a compound of formula (VII)

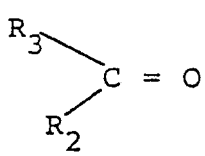

(VII)

wherein $R_2$ and $R_3$ are as defined in relation to formula (I).

The reaction is suitably carried out in an inert organic solvent such as toluene or an alcohol such as ethanol, at an elevated temperature of from 60°C to 150°C, preferably under reflux. When a water immiscible solvent such as toluene is used, water formed during the reaction is preferably removed using a trap.

The present invention also provides a process for preparing a compound of formula (I) or (IA) wherein $R_5$ is hydrogen by reacting the compound of formula (V) with a compound of formula (VII) under reductive conditions.

For example the reaction may be carried out in the presence of a hydride reducing agent such as $NaBH_3CN$ at neutral pH in an organic solvent such as methanol. Moderate temperatures of for example 0°C-30°C suitably 25°C can be employed. The reaction can be carried out over an extended period of time, for example for 18 hours or more.

If hydrogen in the presence of a catalyst such as platinum or nickel is used as the reducing agent, elevated temperatures of for example from 20°C to 100°C and/or elevated pressures suitably of up to 10 atmospheres can be employed.

The present invention further provides a process for preparing a compound of formula (I) or -(IA) by reaction of a compound of formula (VIII)

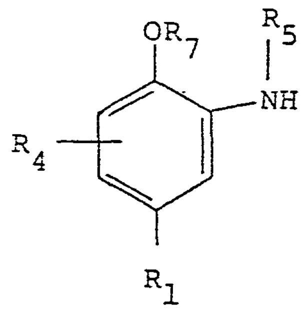

(VIII)

wherein $R_1$, $R_4$, and $R_5$ are as defined in relation to formula (I); and $R_7$ is hydrogen or a hydroxy protecting group;

with a compound of formula (IV) as hereinbefore defined and thereafter if necessary, removing any hydroxy protecting group $R_7$.

Suitable hydroxy protecting groups $R_7$ include benzyl optionally substituted with groups such as nitro or methoxy; and $SO_2$ $CH_3$.

The reaction is suitably carried out in an inert organic solvent such as N,N-dimethylformamide in the presence of a base such as potassium hydroxide or sodium hydride. Elevated temperatures for example of from 25°C to 160°C are preferably employed.

Alternatively the reaction is carried out in aqueous solution in the presence of a base such as sodium hydroxide at ambient temperature.

Compounds of formula (VII) and (VIII) are either known compounds or can be produced from known compounds by conventional methods.

The present invention additionally provides a process for preparing compounds of formula (I) and (IA) wherein $R_3$ is hydrogen and $R_5$ is hydrogen or straight or branched $C_{1-6}$ alkyl by reduction of a compound of formula (IX)

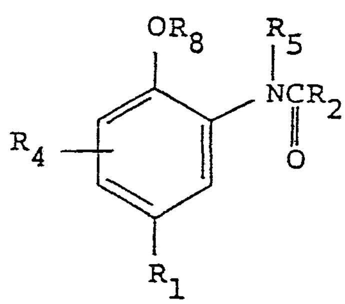

(IX)

wherein $R_1$, $R_2$, $R_4$, and $R_5$ are as defined in relation to formula (I) and $R_8$ is hydrogen or a group reducible to hydrogen.

Suitably $R_8$ is hydrogen, benzoyl or benzyl optionally substituted with groups such as nitro or methoxy.

The reaction can be carried out in the presence of a hydride reducing agent. The hydride reducing agent will be selected to take into account any other reducible group present. Examples of hydride reducing agents which may be employed include $BH_3$-THF (boranetetrahydrofuran), lithium aluminium hydride or boranedimethylsulphide.

The reaction is suitably carried out in an inert solvent such as ether or tetrahydrofuran at a temperature of from 0°C to 80°C.

Compounds of formula (IX) can be prepared from a compound of formula (VIII) as hereinbefore defined wherein $R_7$ is hydrogen and $R_5$ is hydrogen or straight or branched $C_{1-6}$ alkyl by conventional methods. For example, a compound of formula - (VIII) wherein $R_7$ is hydrogen and $R_5$ is hydrogen or straight or branched $C_{1-6}$ alkyl can be reacted with either:

a) a compound of formula (X)

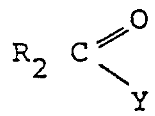

(X)

wherein $R_2$ is as defined in relation to formula (I) and Y is halogen such as chlorine; in the presence of a base such as $Et_3N$ or pyridine; or

b) a compound of formula (XI)

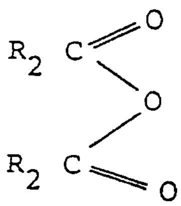

(XI)

wherein $R_2$ is as defined in relation to formula (I), under conventional conditions for the reaction of an anhydride with an amine, or

c) a compound of formula (XII)

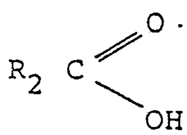

(XII)

wherein $R_2$ is as defined in relation to formula (I), in the presence of a conventional condensing agent such as dicyclohexylcarbodiimide(DCCI), diethyl azodicarboxylate/ triphenylphosphine.

Compounds of formula (X), (XI) and (XII) are known compounds or can be prepared from known compounds by conventional methods.

The present invention also provides a process for preparing a compound of formula (I) or (IA) wherein $R_5$ is $COR_6$ by reaction of a compound of formula (XIII)

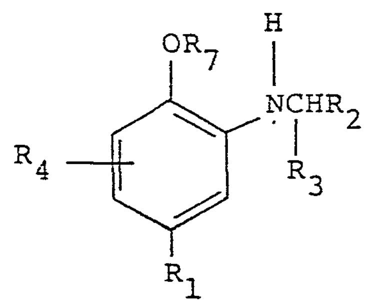

(XIII)

wherein $R_1$, $R_2$, $R_3$ and $R_4$ are as defined in relation to formula (I) and $R_7$ is as defined in relation to formula (VIII) with a compound of formula (X) or a compound of formula (XI) as hereinbefore defined, and thereafter if necessary removing any hydroxy protecting group $R_7$;

Suitable reaction conditions are those conventionally employed in acylation reactions.

The present invention additionally provides a process for preparing a compound of formula (I) or (IA) wherein $R_5$ is hydrogen by reaction of a compound of formula (XIV)

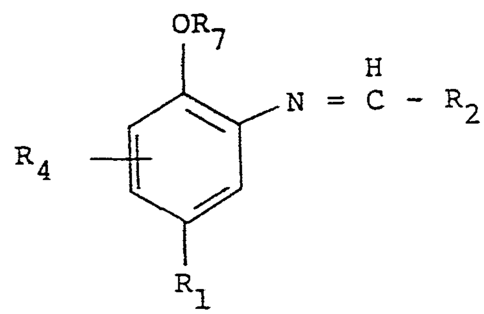

(XIV)

wherein $R_1$, $R_2$ and $R_4$ are as defined in relation to formula (I) and $R_7$ is as defined in relation to formula (VIII); with a compound of formula (XV)

$R_3$ -Z (XV)

wherein $R_3$ is as defined in relation to formula (I) and Z is a metal ion or metal ion complex; and thereafter if necessary removing any hydroxy protecting group $R_7$;

Suitable examples of Z include magnesium halide ion such as $MgBr^+$, and alkali metals such as lithium.

The reaction is suitably carried out in an inert organic solvent such as dry ether or benzene under reflux.

Compounds of formula (XIV) and (XV) are either known compounds or can be prepared from known compounds by known methods.

The compound of formula (I) or (IA) prepared by any of the above described processes of the invention may be obtained where appropriate in the form of a salt and/or solvate, preferably a pharmaceutically acceptable salt or solvate. Salts or solvates which are not pharmaceutically acceptable may be converted into salts or solvates which are pharmaceutically acceptable or into compounds per se. Compounds of formulae (I) or (IA) may be converted into other such compounds by suitable interconversion of substituents.

The compounds of formula (I) and (IA) are indicated as active therapeutic agents by their inhibition of 5-lipoxygenase.

Accordingly the present invention also provides a pharmaceutically acceptable compound of the formula (I) and (IA), or a pharmaceutically acceptable salt or solvate thereof, for use in treatment of the human or animal body, particularly for the treatment or prophylaxis of allergic diseases, such as asthma, hayfever, rhinitis or allergic eczema, or for treatment of inflammatory conditions such as arthritis and psoriasis.

The present invention also provides a pharmaceutical composition comprising a pharmaceutically acceptable compound of the formula (I) and (IA), or a pharmaceutically acceptable salt or solvate thereof, in admixture or conjunction with a pharmaceutically acceptable carrier.

Preferably, a pharmaceutical composition of the invention is in unit dosage form and in a form adapted for use in the medical or veterinarial fields. For example, such preparations may be in a pack form accompanied by written or printed instructions for use as an agent in the treatment of prophylaxis of any of the disorders mentioned above.

The suitable dosage range for the compounds of the invention may vary from compound to compound and may depend on the condition to be treated. It will also depend, interalia, upon the relation of potency to absorbability and the mode of administration chosen. The compound or composition of the invention may be formulated for administration by any route, the preferred route depending upon the disorder for which treatment is required, and is preferably in unit dosage form or in a form that a human patient may administer to himself in a single dosage. Advantageously, the composition is suitable for oral, rectal, topical, parenteral, intravenous or intramuscular administration or through the respiratory tract. Preparations may be designed to give slow release of the active ingredient.

Compositions may, for example, be in the form of tablets, capsules, sachets, vials, powders, granules, lozenges, reconstitutable powders, or liquid preparations, for example solutions or suspensions, or suppositories. Compositions which are especially suitable for administration to the respiratory tract and for topical administration are discussed in more detail below.

The compositions, for example those suitable for oral administration, may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable setting agents such as sodium lauryl sulphate.

Solid compositions may be obtained by conventional methods of blending, filling tabletting or the like. Repeated blending operations may be used to distribute the active agent throughout those compositions employing large quantities of fillers. When the composition is in the form of a tablet, powder, or lozenge, any carrier suitable for formulating solid pharmaceutical compositions may be used, examples being magnesium stearate, starch, glucose, lactose, sucrose, rice flour and chalk. Tablets may be coated according to methods well known in normal pharmaceutical practice, in particular with an enteric coating. The composition may also be in the form of an ingestible capsule, for example of gelatin containing the compound, if desired with a carrier or other excipients.

Compositions for oral administration as liquids may be in the form of, for example, emulsions, syrups, or elixirs, or may be presented as a dry product for reconstitution with water or other suitable vehicle before use. Such liquid compositions may contain conventional additives such as suspending agents, for example sorbitol, syrup, methyl cellulose, gelatin, hydroxyethylcellulose carboxymethylcellulose, aluminium stearate gel, hydrogenated edible fats; emulsifying agents, for example lecithin, sorbitan monooleate, or acacia; aqueous or non-aqueous vehicles, which include edible oils, for example almond oil, fractionated coconut oil, oily esters, for example esters of glycerine, or propylene glycol, or ethyl alcohol, glycerine, water or normal saline; preservatives, for example methyl or propyl p-hydroxybenzoate or sorbic acid; and if desired conventional flavouring or colouring agents.

The compounds of this invention may also be administered by a non-oral route. In accordance with routine pharmaceutical procedure, the compositions may be formulated, for example for rectal administration as a suppository or for presentation in an injectable form in an aqueous or non-aqueous solution, suspension or emulsion in a pharmaceutically acceptable liquid, e.g. sterile pyrogen-free water or a parenterally acceptable oil or a mixture of liquids, which may contain bacteriostatic agents, anti-oxidants or other preservatives, buffers or solutes to render the solution isotonic with the blood, thickening agents, suspending agents or other pharmaceutically acceptable additives. Such forms will be presented in unit dose form such as ampoules or disposable injection devices or in multi-dose forms such as a bottle from which the appropriate dose may be withdrawn or a solid form or concentrate which can be used to prepare an injectable formulation.

Compositions of this invention may also suitably be presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns. Where appropriate small amounts of other anti-asthmatics and bronchodilators, for example sympathomimetic amines such as isoprenaline, isoetharine, sal-

butamol, phenylephrine and ephedrine; xanthine derivatives such as theophylline and aminophylline and corticosteroids such as prednisolone and adrenal stimulants such as ACTH may be included.

For topical administration, compositions may be presented as an ointment, cream, lotion, gel, gel stick, spray, aerosol, or skin paint.

By way of example, in any of the preceding formulations a suitable dosage unit might contain 0.01 to 500 mgs of active ingredient, more suitably 1 to 500 mgs via the oral route, 0.01 to 10mgs via inhalation. The effective dose of compound depends on the particular compound employed, the condition of the patient and on the frequency and route of administration, but in general is in the range of from 0.001 mg/kg/day to 100 mg/kg/day inclusive of the patient's body weight. For use in treatment of inflammatory disorders a composition of the invention will preferably be in a form suitable for oral administration, for example a tablet or capsule or a sachet containing reconstitutable powder. A unit dose will generally contain from 20 to 1000 mg and preferably will contain from 30 to 500 mg, in particular 50, 100, 150, 200, 250, 300, 350, 400, 450, or 500 mg. The composition may be administered once or more times a day for example 2, 3 or 4 times daily, and the total daily dose for a 70 kg adult will normally be in the range 100 to 3000 mg. Alternatively the unit dose will contain from 2 to 20 mg of active ingredient and be administered in multiples, if desired, to give the preceding daily dose.

Within the above mentioned dosage ranges, no adverse toxicological effects are indicated with the compounds of the invention.

The present invention also provides a method for treating allergic diseases or inflammatory conditions in human or non-human animals which comprises administering to the sufferer an effective non-toxic amount of a compound according to formula (I) or (IA) or a pharmaceutically acceptable salt or solvate thereof.

Also included in the present invention is the use of a compound of formula (I) or (IA), or a pharmaceutically acceptable salt or solvate thereof, in the manufacture of a medicament for treatment of the above disorders.

Compounds of this invention, their preparation and their biological activity are illustrated in the following Examples and Pharmacological Data. The compounds set out in Tables I and II were prepared by methods analogous to those described in Examples 1 to 6.

Example 1

5-Methyl-2(3H)-benzoxazolinone

2-Amino-4-methylphenol (6.5g, 53mmol) and urea (4g,67mmol) were heated together at 160°C for 2½ hours and cooled. The resulting solid was partitioned between 2Nag.hydrochloric acid and diethyl ether. The organic solution was dried ($MgSO_4$), concentrated in vacuo and the resulting grey solid purified by column chromatography on silica eluting with chloroform to afford the title compound (8.84g, 87%) as a pale pink solid mp. 131-3°C.

$\nu_{max}$(mull) 3350, 1790, 1740cm$^{-1}$.

δ($CDCl_3$): 2.27 (3H, s, $CH_3$), 6.70-7.10 (3H, s, aromatic), 10.00 (1H, s, NH)

Found: C: 64.60; H: 4.60; N:9.30%. $C_8H_7NO$ requires C: 64.41; H: 4.73; N: 9.39%.

Example 2

3-Phenylmethyl-5-methyl-2-benzoxazolinone

5-Methyl-2(3H)-benzoxazolinone (1g, 6.7mmol) and benzylbromide (1.26g, 7.35mmol) in EtOH - (40ml) were heated together under reflux in the presence of potassium hydroxide (0.42g, 7.5mmol) in $H_2O$ (4ml) for 80 mins. The mixture was allowed to cool, the resulting solid was collected by filtration and recrystallized from EtOH to afford the title compound (1.42g, 89%) as a white solid. mp 123-4°C.

$\nu_{max}$(mull) 1775, 1760cm$^{-1}$.

δ(DMSO): 2.30 (3H, s, $CH_3$), 5.02 (2H, s, $CH_2$), 6.93 (1H, d.d, J= 1Hz, 8Hz, H-6), 7.05 (1H, d, J = 1Hz, H-4), 7.23 (1H, d, 8Hz, H-7), 7.27-7.40 (5H, m, aromatic).

Found: C: 75.59; H: 5.43; N: 5.91%. $C_{15}H_{13}NO_2$ requires C: 75.29; H: 5.47; N:5.86.

Example 3

2-Phenylmethylamino-4-methylphenol

3-Phenylmethyl-5-methyl-2-benzoxazolinone - (5g, 21mmol) and potassium hydroxide (7g. 0.126mol) in MeOH (40ml) were heated together under reflux for 1 hour. The solvent was removed in vacuo, the residue taken up in $H_2O$ and neutralised with aqueous sodium bicarbonate solution. The resulting solid was collected by filtration and further

purified by column chromatography on silica eluting with chloroform to afford the title compound (1.5g, 38%) as an off white solid. This solid was taken up in diethyl ether and converted to its hydrochloride salt with HCl gas which could be recrystallised from EtOH: $Et_2O$. mp. 183-4°C.

$\nu_{max}$ (mull) 3120, 2740, 1565cm$^{-1}$,

δ(DMSO): 2.13 (3H, s, $CH_3$), 4.42 (2H, s, $CH_2$), 7.02 (2H, s, H-4,6), 7.22 (1H, s, H-7), 7.20-7.50 (5H, m, aromatic), 9.20-11.60 (3H, br s, OH, $NH_2^{\oplus}$, exchangeable).

Found: C: 67.13; H: 6.47; N: 5.60%. $C_{14}H_{16}ClNO$ requires C: 67.33; H: 6.48; N: 5.61%.

Example 4

2-(4-Cyanobenzylidene)amino-4-methylphenol

2-Amino-4-methylphenol (1.9g, 15.3mmol) and 4-cyanobenzaldehyde (2g, 15.3mmol) were heated together under reflux in toluene (2.5ml) for 4 hours removing the water produced with a Dean and Stork trap. The solution was allowed to cool, the resulting solid collected by filtration, washed with toluene and recrystallised from $Et_2O$. The title compound (3.09g, 86%) was obtained as a yellow solid m.p. 152-3°C.

$\nu_{max}$ (mull) 3400, 2220, 1620, 1500, 1245cm$^{-1}$.

δ(DMSO): 2.25 (3H, s, $CH_3$), 6.80-7.17 (3H, m, aromatic), 7.95 (2H, d, J= 8Hz, aromatic), 8.25 (2H, d, J = 8Hz, aromatic), 8.87 (1H, s, CH=), 8.97 - (1H, s, OH, exchangeable).

Found: C: 76.31; H: 5.15; N: 11.92%. $C_{15}H_{12}N_2O$ requires C: 76.25; H: 5.12; N: 11.86%.

Example 5

2-[(4-Cyanophenyl)methyl]amino-4-methylphenol

A suspension of 2-(4-cyanobenzylidene)amino-4-methylphenol (1.8g, 7.6mmol) in EtOH (20ml) at room temperature was acidified to pH 1 with ethanolic hydrogen chloride and treated portionwise with sodium cyanoborohydride (0.5g, 7.6mmol) over 10 mins. The reaction mixture was maintained at pH 1-2 by the addition of ethanolic hydrogen chloride. After 1 hour the mixture was poured onto ice, neutralised with aqueous sodium bicarbonate solution and the resulting solid collected by filtration. Recrystallisation from $Et_2O$ afforded the title compound (1.14g, 63%) as a white solid mp 137-138°C.

$\nu_{max}$ (mull) 3500-2800 (br), 3330, 2225, 1520cm$^{-1}$.

δ(DMSO): 2.07 (3H, s, $CH_3$), 4.40 (2H, d, J = 6Hz, $CH_2$), 5.42 (1H, t, J = 6Hz, NH, exchangeable), 6.18 (1H, d, J = 1.5Hz, H-3), 6.24 (1H, d.d, J = 1.5Hz. 8Hz. H-5), 6.62 (1H, d, J = 8Hz, H-6), 7.55 - (2H, d, J = 8Hz, aromatic), 7.78 (2H, d, J = 8Hz, aromatic), 9.08 (1H, s, OH, exchangeable).

Found: C: 75.63; H: 6.04; N: 11.86%. $C_{15}H_{14}N_2O$ requires C: 75.60; H: 5.92; N: 11.76%.

Example 6

2-[(4-Methylphenyl)methyl]amino-4-methylphenol

2-Amino-4-methylphenol (4.93g, 40mmol) and 4-methylbenzaldehyde (4.81g, 40mmol) in methanol (50ml) were stirred with sodium cyanoborohydride (2.51g, 40mmol) at 25°C for 18 hours. The reaction mixture was poured into $H_2O$ - (250ml), extracted with $CH_2Cl_2$ (3 × 50 ml) and the combined extracts dried ($MgSO_4$). Concentration in vacuo afford a red oil which was purified by column chromatography on silica eluting with $CH_2Cl_2$. The major component was treated with ethanolic hydrogen chloride, the ethanol was removed and the resulting solid recrystallised from ethyl acetate/petroleum ether (40-60°C) to afford the title compound as its hydrochloride salt (5.07g, 48%) m.p. 172-4°C.

$\nu_{max}$ (mull) 3100, 2740, 1630, 1565, 1520 cm$^{-1}$

δ(DMSO) 2.16 (3H, s, $CH_3$), 2.30 (3H, s, $CH_3$), 4.39 - (2H, s, $CH_2$), 7.00-7.50 (7H, m, aromatic), 10.00-11.00 (3H, br s, $NH_2^{\oplus}$, OH, exchangeable).

Found: C: 68.36; H: 7.04; N: 5.45; Cl: 13.48%. $C_{15}H_{18}ClNO$ requires; C: 68.30; H: 6.88; N: 5.31; Cl: 13.44%.

TABLE I

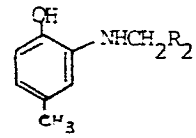

| Example | $R_2$ | Found = F Calculated = C | C | H | N | Halogen | S | m.p. (°C) |
|---|---|---|---|---|---|---|---|---|
| 7 | 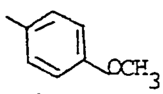 | F<br>C | 73.88<br>74.05 | 7.08<br>7.05 | 5.72<br>5.76 | | | 143-4 |
| 8 | 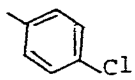 .HCl | F<br>C | 59.15<br>59.17 | 5.34<br>5.32 | 5.16<br>4.93 | 25.90<br>24.93 | | 168-70 |
| 9 | 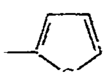 .HCl | F<br>C | 60.41<br>60.13 | 6.05<br>5.89 | 5.88<br>5.84 | 15.24<br>14.79 | | 160-1 |
| 10 | 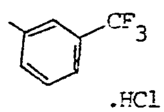 .HCl | F<br>C | 56.76<br>56.70 | 4.88<br>4.76 | 4.62<br>4.41 | 11.17<br>11.16 | | 189-91 |
| 11 | 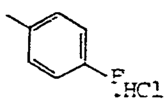 .HCl | F<br>C | 62.92<br>62.80 | 5.76<br>5.65 | 5.23<br>5.23 | 13.11<br>13.24 | | 180-2 |
| 12 | 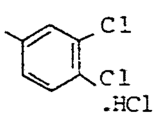 .HCl | F<br>C | 53.03<br>52.77 | 4.46<br>4.43 | 4.42<br>4.23 | 33.33<br>33.38 | | 183-5 |
| 13 | 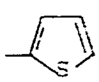 .HCl | F<br>C | 56.52<br>56.35 | 5.44<br>5.52 | 5.39<br>5.48 | 14.18<br>[illegible] | 12.46<br>12.51 | 163-5 |
| 14 | 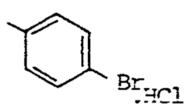 .HCl | F<br>C | 51.23<br>51.16 | 4.54<br>4.60 | 4.46<br>4.26 | 34.64<br>35.10 | | 172-4 |
| 15 | 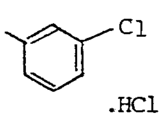 .HCl | F<br>C | 59.34<br>59.17 | 5.48<br>5.32 | 5.00<br>4.93 | 25.17<br>24.95 | | 200-2 |
| 16 | 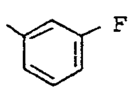 .HCl | F<br>C | 62.72<br>62.80 | 5.77<br>5.65 | 5.16<br>5.23 | 13.24<br>13.24 | | 188-9 |

55

Example 17

2 -(α -Methylbenzyl)amino -4 -methylphenol

This compound was prepared by methods analogous to those described in examples 4-6 and characterised as its hydrochloride salt. m.p. 186-7°C (EtOH).

Found: C : 68.14; H : 6.84; N : 5.30; Cl : 13.22%. $C_{15}H_{18}ClNO$ requires C : 68.30; H : 6.88; N : 5.31; Cl : 13.44%.

Example 18

2 -Phenylmethylamino -4 -methylphenol via 2 -Benzolyamino -4 -methylphenol

2 -Benzoylamino -4 -methylphenol (520mg, 2.3mmol), prepared according to the method of Seha [Helv. Chim. Acta. 1980, 63, 916), in dry THF (50ml) was treated with lithium aluminium hydride - (260mg, 6.5mmol) at room temperature,with stirring under nitrogen for 4 hours $H_2O$ (0.1ml), 10% aq. NaOH (0.1ml)and $H_2O$ (1ml) were added successively to the reaction mixture, the resulting solid removed by filtration, washed with $Et_2O$ (2 x 25ml) and the combined filtrates concentrated. The resulting solid was taken up into 2N aq HCl solution, the solution adjusted to pH 8 with saturated aq. $NaHCO_3$ solution and extracted with $CHCl_3$ - (3x50ml). The combined extracts were dried - ($MgSO_4$), concentrated in vacuo and the resulting solid purified by column chromatography on silica eluting with $CH_2Cl_2$ to afford the title compound, in a modest yield, which exhibited identical physical and spectroscopic properties to an authentic sample.

Example 19

2 -Phenylmethylamino -4 -methylphenol via 2 -Benzoylamino -4 -methylphenol O-benzoate

2 -Benzoylamino -4 -methylphenol O -benzoate (600mg, 1.81mmol), prepared from 2 -amino -4 -methylphenol and benzoyl chloride, in dry THF - (50ml) was treated with lithium aluminium hydride - (300mg, 7.9mmol) at room temperature with stirring under nitrogen for 3 hours. Saturated aq. $NaHCO_3$ solution (20ml) was added slowly and the two layers separated. The aqueous phase was extracted with $Et_2O$ (3 x 20ml), the combined organic phases washed with $H_2O$ (20ml), dried ($MgSO_4$) and concentrated in vacuo. Purification of the resulting solid by column chromatography on silica eluting with $CH_2Cl_2$ afforded the title compound (270mg. 70%) which, as its hydrochloride salt, showed identical physical and spectroscopic properties to an authentic sample.

TABLE I - continued

| | | | | | | | | |
|---|---|---|---|---|---|---|---|---|
| 25 | 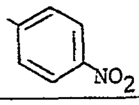 .HCl | F | 59.95 | 5.13 | 9.48 | 12.38 | | 180-1 |
| | | C | 57.05 | 5.13 | 9.51 | 12.03 | | |
| 26 | 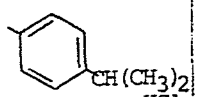 .HCl | F | 69.79 | 7.66 | 4.69 | 12.47 | | 159-61 |
| | | C | 69.97 | 7.60 | 4.80 | 12.15 | | |
| 27 | 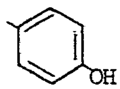 .HCl | F | 63.13 | 5.96 | 5.24 | 13.23 | | 148-50 |
| | | C | 63.27 | 6.07 | 5.27 | 13.34 | | |
| 28 | $CH_2C_6H_5$ | F | 69.14 | 6.84 | 5.24 | | | 153-4 |
| | | C | 68.30 | 6.88 | 5.31 | | | |

TABLE II

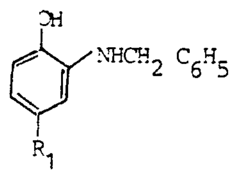

| Example | $R_1$ | Elemental Analysis (%) Found = F Calculated = C | C | H | N | Halogen | m.p. (°C) |
|---|---|---|---|---|---|---|---|
| 20 | $CH(CH_3)_2$ .HCl | F<br>C | 69.27<br>69.18 | 7.26<br>7.26 | 5.05<br>5.04 | 12.58<br>12.76 | 180-1 |
| 21 | $C(CH_3)_3$ .HCl | F<br>C | 69.96<br>69.97 | 7.44<br>7.60 | 4.84<br>4.80 | 11.70<br>12.15 | 86-9 |
| 22 | Cl .HCl | F<br>C | 57.67<br>57.79 | 5.13<br>4.85 | 5.03<br>5.19 | 26.69<br>26.25 | 181-3 |
| 23 | Br .HCl | F<br>C | 49.46<br>49.63 | 4.25<br>4.16 | 4.63<br>4.45 | 35.93<br>36.67 | 186-7 |
| 24 | $CH_2CH_3$ .HCl | F<br>C | 68.31<br>68.30 | 6.90<br>6.88 | 5.11<br>5.31 | 13.38<br>13.44 | 180-1 |

Example 29

4-Benzoyl-6-(Phenylmethylamino)resorcinol hydrochloride

This compound was prepared from 4-benzoyl-6-aminoresorcinol by methods analogous to those described in examples 4-6. m.p. 209-211°C.

Found: C:67.59; H:5.26; N:3.97%. $C_{20}H_{18}ClNO_3$ requires C:67.51; H:5.10; N:3.94%.

Example 30

2-Dibenzylamino-4-methylphenol

A solution of 2-amino-4-methylphenol (12.32g, 0.1mol) in 2N aq. NaOH (50ml) was stirred with benzyl bromide (17.1g, 0.1mol) at room temperature for 18 hours. The mixture was extracted with $Et_2O$ (2 x 100ml), the combined extracts washed successively with 2N aq.NaOH solution (50ml) and $H_2O$ (50ml) and dried ($MgSO_4$). Concentration in vacuo afforded a red oil which was purified by column chromatography on silica eluting with hexane: $Et_2O$ (4:1,v/v). The major component was dissolved in $Et_2O$ and treated with etherial hydrogen chloride, the resulting precipitate was collected by filtration, washed with $Et_2O$ and recrystallised from EtOH to afford the title compound as its hydrochloride salt (11.81g, 69%) m.p. 197-200°C.

Found: C:73.29; H:6.44; N:4.02; Cl:10.06%. $C_{21}H_{22}ClNO$. $0.25H_2O$ required C:73.24; H:6.58; N:4.06; Cl:10.29%.

The minor component from the chromatographic separation was taken up into $Et_2O$ and treated with etherial hydrogen chloride to afford a white solid which was collected by filtration and recrystallised from EtOH. This compound was shown to be 2-phenylmethylamino-4-methylphenol hydrochloride (1.55g, 7%) by comparison with a previously prepared sample. m.p. 181-2°C.

Found: C:67.14; H:6.56; N:5.32; Cl:14.22. $C_{14}H_{16}ClNO$ requires: C:67.33; H:6.48; N: 5.61; Cl:14.20.

PHARMACOLOGICAL DATA

1) RBL-1 5-LIPOXYGENASE SCREEN

5-Lipoxygenase enzyme was prepared as a 10,000g supernatant from RBL-1 cells by the method of Jakschik (Jakschik, B.A., F.F. Sun, L.M. Lee, and M.M. Steinhoff, 1980, Biochem. Biophys. Res. Comm. 95, 103). The 10,000g supernatant was diluted with homogenization buffer to the equivalent of 1.5 -2.5 x $10^7$ cells. $ml.^{-1}$ and made 2mM with respect to $CaCl_2$. Aliquots of 0.5ml were then dispensed into tubes, and incubated at 29°C with 5ul ethanol or compound in ethanol at the desired concentration for 2min. Then [1-$^{14}$C] arachidonic acid was added in buffer to give a final concentration of 6.3uM and 0.2uCi per incubation, and the reaction continued at 29°C for 2 min. The reaction was terminated by adding 1ml of acetone and cooling on ice, 0.5ml of ice-cold saline and 100ul of 2N formic acid were added, and the mixture was extracted with 2 x 2ml of chloroform. The extract was stored under $N_2$ at -20°C until analysis by chromatography. Activity was measured as the percentage of total radioactivity found in 5-HETE and 5,12-diHETE, and inhibition calculated as the decrease information of the sum of these two species in compound-treated incubates relative to control incubations.

Biological Results



| Example No | % Inhibition of RBL-1 5-lipoxygenase (concentration in brackets; uM) | |
|---|---|---|
| 3 | 95.4 | (20) |
| 5 | 42.8 | (0.05) |
| 6 | 93.4 | (0.05) |
| 7 | 61.7 | (0.05) |
| 8 | 91.4 | (0.05) |
| 9 | 31.4 | (0.05) |
| 10 | 89.5 | (0.05) |
| 11 | 93.2 | (0.05) |
| 12 | 73.7 | (0.05) |
| 13 | 49.7 | (0.05) |
| 14 | 64.2 | (0.05) |
| 15 | 85 | (0.05) |
| 16 | 76 | (0.05) |
| 17 | 34 | (0.05) |
| 20 | 77 | (0.05) |
| 21 | 35 | (0.05) |
| 22 | 81 | (0.05) |
| 23 | 98 | (20) |
| 24 | 20 | (0.05) |
| 25 | 80 | (0.05) |
| 26 | 80 | (0.05) |
| 27 | 12 | (0.05) |
| 28 | 86 | (0.05) |
| 29 | 94 | (1) |
| 30 | 91 | (1) |

## Claims

1. A pharmaceutical composition comprising a compound of formula (I)

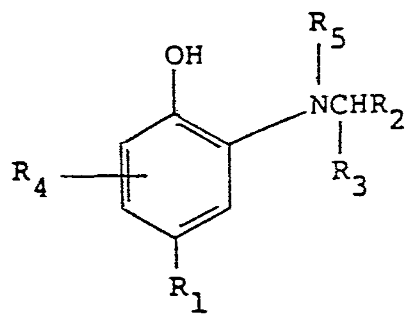

(I)

or a pharmaceutically acceptable salt or solvate thereof,

wherein $R_1$ is straight or branched $C_{1-6}$ alkyl, halo, nitro, cyano, hydroxy, or $COR_6$ wherein $R_6$ is straight or branched $C_{1-6}$ alkyl or aryl,

$R_2$ is substituted to unsubstituted aryl, aralkyl or heteroaryl;

$R_3$ is hydrogen or straight or branched $C_{1-6}$alkyl;

$R_4$ is hydrogen, hydroxy, straight or branched $C_{1-6}$ alkyl, halo, nitro, cyano, $C_{1-6}$ alkoxy, halo $(C_{1-6})$alkyl; amino, carboxylic ester, carboxylic acid, $COR_6$,$SR_6$, $SO_2R_6$, $NHCOR_6$ or $NHR_6$ wherein $R_6$ is as defined above; and

$R_5$ is hydrogen, straight or branched $C_{1-6}$alkyl, substituted or unsubstituted benzyl or $COR_6$ wherein $R_6$ is as defined above;

and a pharmaceutically acceptable carrier.

2. A composition according to claim 1 in which $R_1$ is methyl, <u>iso</u> -propyl, <u>tert</u>-butyl, acetyl, benzyl, bromo or chloro.

3. A composition according to claim 1 or 2 in which $R_2$ is substituted or unsubstituted phenyl, furan, thiophene, imidazole, isoxazole, pyridine or benzyl.

4. A composition according to any one of claims 1, 2 or 3 in which $R_2$ is substituted by one or more groups selected from straight or branched $C_{1-6}$ alkyl, hydroxy, halo, nitro, cyano, $C_{1-6}$ alkoxy, halo - $(C_{1-6})$ alkyl, amino, carboxylic ester, carboxylic acid, $COR_6$, $NHCOR_6$, $NHR_6$, $N(R_6)_2$, $SR_6$, $SOR_6$ or $SO_2R_6$ where $R_6$ is as hereinbefore defined.

5. A composition according to any one of claims 1 to 4 in which $R_3$ is hydrogen or methyl.

6. 6. A composition according to any one of claims 1 to 5 in which $R_4$ is hydrogen, trifluoromethyl, methyl, <u>tert</u> butyl or benzoyl.

7. A composition according to any one of claims 1 to 6 in which $R_5$ is $-COCH_3$ or hydrogen.

8. A composition according to any one of claims 1 to 7 which comprises a compound of formula (I) in which

$R_1$ is methyl, ethyl, <u>iso</u>-propyl, <u>tert</u> -butyl, benzoyl, chloro or bromo,

$R_2$ is phenyl, benzyl, furyl or thienyl,

optionally substituted by methyl, <u>iso</u>-propyl, cyano, methoxy, chloro, bromo, fluoro,

trifluoromethyl, hydroxy or nitro,

$R_3$ is hydrogen or methyl,

$R_4$ is hydrogen or hydroxy, and

$R_5$ is hydrogen or benzyl.

9. A composition according to claim 1 in which the compound of formula (I) is selected from

2-phenylmethylamino-4-chlorophenol,

2-phenylmethylamino-4-methylphenol,

and pharmaceutically acceptable salts and solvates thereof.

10. A compound of formula (IA), or salt or solvate thereof:

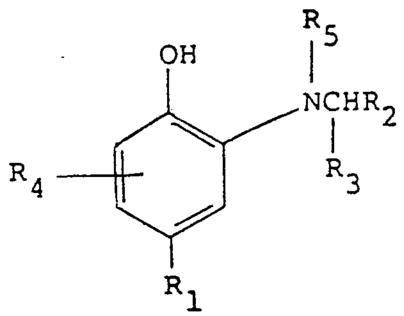

(IA)

wherein $R_1$, $R_2$, $R_3$, $R_4$, and $R_5$ are as defined in relation to formula (I) in any one of claims 1 to 8, provided that when $R_1$ is methyl or chloro, $R_2$ is phenyl, $R_3$ is hydrogen and $R_4$ is hydrogen, $R_5$ is other than hydrogen.

11. A compound according to claim 10 which is selected from:

2-[(4-cyanophenyl)methyl]amino-4-methylphenol;

2-[(4-methylphenyl)methyl]amino-4-methylphenol;

2-[(4-methoxyphenyl)methyl]amino-4-methylphenol;

2-[(4-chlorophenyl)methyl]amino-4-methylphenol;

2-[(2-furyl)methyl]amino-4-methylphenol;

2-[(3-trifluoromethylphenyl)methyl]amino-4-methylphenol;

2-[(4-fluorophenyl)methyl]amino-4-methyl phenol;

2-[(3,4 dichlorophenyl)methyl]amino-4-methyl phenol;

2-[(thien -2-yl)methyl]amino-4-methyl phenol;

2-[(4-bromophenyl)methyl]amino-4-methyl phenol;

2-[(3-fluorophenyl)methyl]amino-4-methyl phenol;

2-phenylmethylamino-4-iso-propylphenol;

2-phenylmethylamino-4-tert-butylphenol;

4-benzoyl-6-(phenylmethylamino) resorcinol;

2-dibenzylamino-4-methyl phenol;

2-phenylmethylamino-4-chlorophenol;

2-phenylmethylamino-4-bromophenol;

2-phenylmethylamino-4-ethylphenol;

2-[)4-nitrophenyl]methyl]amino-4-methylphenol 2-]-(4-iso-propylphenyl)methyl]amino-4-methylphenol;

2-[(4-hydroxyphenyl)methyl]amino-4-methylphenol;

2-phenylethylamino-4-methylphenol;

and salts and solvates thereof.

12. A compound according to claim 10 or 11 in pharmaceutically acceptable and/or substantially pure form.

13. A process for preparing a compound of formula (I) as defined in claim 1

(a) when $R_5$ is hydrogen, by hydrolysis of a compound of formula (II)

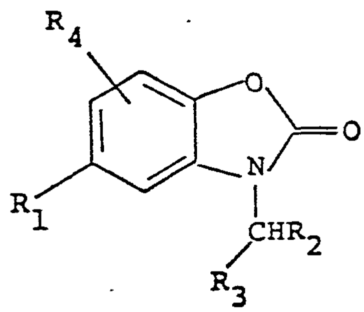

(II)

wherein $R_1$, $R_2$, $R_3$ and $R_4$ are as defined in relation to formula (I),

(b) when $R_5$ is hydrogen, by reduction of a compound of formula (VI)

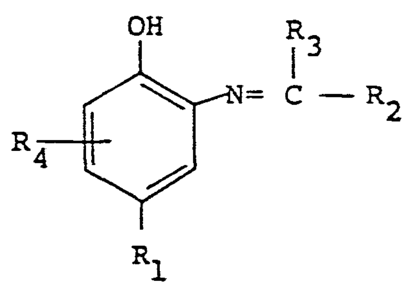

(VI)

wherein, $R_1$, $R_2$ $R_3$ and $R_4$ are as defined in relation to formula (I),

(c) when $R_5$ is hydrogen by reacting the compound of formula (V)

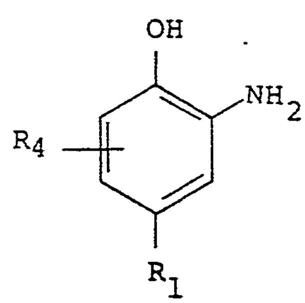

with a compound of formula (VII)

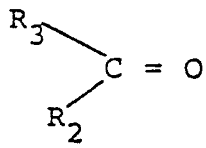

under reductive conditions,

(d) by reaction of a compound of formula (VIII)

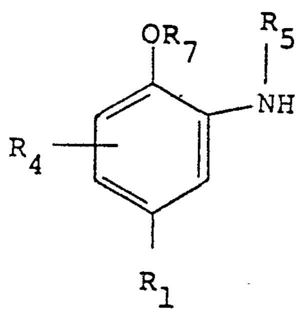

(VIII)

wherein $R_1$, $R_4$ and $R_5$ are as defined in relation to formula (I); and $R_7$ is hydrogen or a hydroxy protecting group;

with a compound of formula (IV)

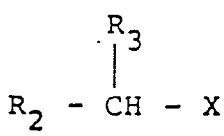

and thereafter if necessary, removing any hydroxy protecting group $R_7$,

(e) when $R_3$ is hydrogen and $R_5$ is hydrogen or straight or branched $C_{1-6}$ alkyl, by reduction of a compound of formula (IX)

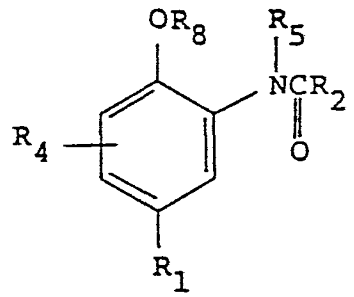

(IX)

wherein $R_1$, $R_2$, $R_4$ and $R_5$ are as defined in relation to formula (I) and $R_8$ is hydrogen or a group reducible to hydrogen,

(f) when $R_5$ is $COR_6$, by reaction of a compound of formula (XIII)

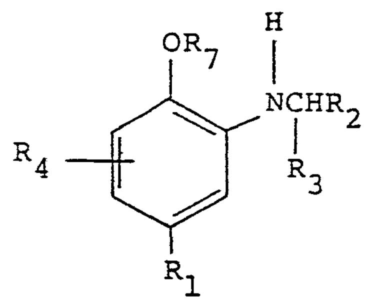

(XIII)

wherein $R_1$, $R_2$, $R_3$ and $R_4$ are as defined in relation to formula (I) and $R_7$ is hydrogen or a hydroxy protecting group,

with a compound of formula (X)

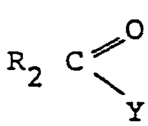

or a compound of formula (XI)

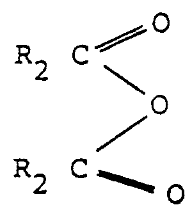

and thereafter ifnecessary removing any hydroxy protecting group $R_7$,

(g) when $R_5$ is hydrogen, by reaction of a compound of formula (XIV)

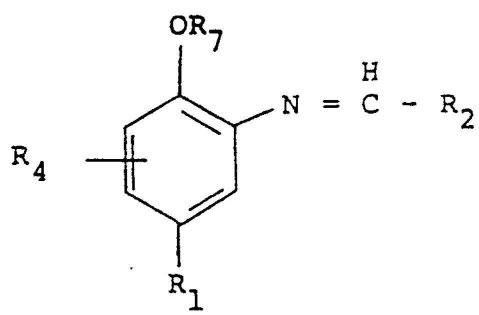

(XIV)

wherein $R_1$, $R_2$ and $R_4$ are as defined in relation to formula (I) and $R_7$ is hydrogen or a hydroxy protecting group;

with a compound of formula (XV)

$R_3$-Z (XV)

wherein $R_3$ is as defined in relation to formula (I) and Z is a metal ion or metal ion complex;

and thereafter if necessary removing any hydroxy protecting group $R_7$, and optionally forming a salt and/or solvate of the obtained compound.

14. A compound of the formula (I) or (IA) as defined in any one of claims 1 to 11, or a pharmaceutically acceptable salt or solvate thereof, for use in treatment of the human or animal body, particularly for the treatment or prophylaxis of allergic diseases, such as asthma, hayfever, rhinitis or allergic eczema, or for treatment of inflammatory conditions such as arthritis and psoriasis.

15. The use of a compound of formula (I) or (IA), as defined in any one of claims 1 to 11, or a pharmaceutically acceptable salt or solvate thereof, in the manufacture of a medicament for treatment of allergic or inflammatory disorders.